# EUROPÄISCHE PATENTSCHRIFT

(11) **EP 1 517 906 B1**
(45) Veröffentlichungstag und Bekanntmachung des Hinweises auf die Patenterteilung: **28.03.2007**
(21) Anmeldenummer: 03760593.8
(22) Anmeldetag: 06.06.2003
(51) Int. Cl.: C07D 405/06

(54) **VERFAHREN ZUR HERSTELLUNG VON 1,2,4-TRIAZOLYLMETHYL-OXIRANEN**
METHOD FOR THE PRODUCTION OF 1,2,4-TRIAZOLYLMETHYL-OXIRANES
PROCEDE DE PRODUCTION DE 1,2,4-TRIAZOLYLMETHYLOXIRANES

(30) Priorität: 24.06.2002 DE 10228196
(43) Veröffentlichungstag der Anmeldung: 30.03.2005
(73) Patentinhaber: BASF Aktiengesellschaft, 67056 Ludwigshafen (DE)
(72) Erfinder: NOACK, Rainer, 04932 Grossthiemig (DE); SANDER, Michael, 67117 Limburgerhof (DE); HENNINGSEN, Michael, 67227 Frankenthal (DE)
(86) Internationale Anmeldenummer: PCT/EP2003/005950
(87) Internationale Veröffentlichungsnummer: WO 2004/000835

(56) Entgegenhaltungen:
- EP-A- 0 618 198
- WO-A-94/02476
- CA-A- 2 051 281
- US-A- 4 906 652

## Beschreibung

Die vorliegende Erfindung betrifft ein Verfahren zur regiospezifischen Herstellung von 1,2,4-Triazol-1-yl-methyl-oxiranen der Formel I, 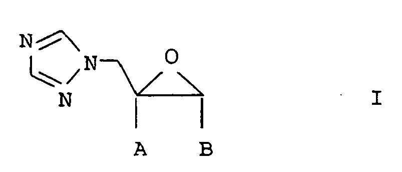 in der A und B gleich oder verschieden sind und unabhängig voneinander C₁-C₄-Alkyl, Phenyl-C₁-C₂-alkyl; C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, wobei man
a) ein Oxiran der Formel II, 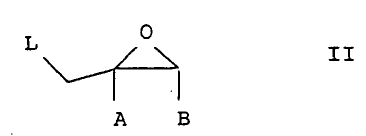 in der A und B die oben genannte Bedeutung besitzen und L für eine nukleophil substituierbare Abgangsgruppe steht, mit 4-Amino-1,2,4-triazol der Formel III 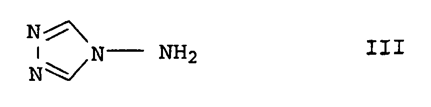 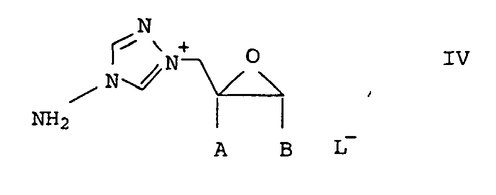 zu 4-Amino-1,2,4-triazoliumsalzen der Formel IV umsetzt und
b) die 4-Amino-1,2,4-triazoliumsalze IV mit Alkalinitriten und Säure oder organischen Nitriten zu 1,2,4-Triazol-1-ylmethyloxiranen der allgemeinen Formel I deaminiert.

4-Aminotriazoliumsalze IV sind Zwischenprodukte für die Herstellung von Azolylmethyloxiranen. Azolylmethyl-oxirane dienen zur Herstellung von fungiziden Mitteln, insbesondere gegen Getreidekrankheiten.

Aus den EP-A 94 564, US 4,906,652, EP-A 330132 und EP-A 334 035 sind Verfahren zur Herstellung von Triazolylmethyloxiranen ausgehend von einem Oxiran der Formel II und 1,2,4-Triazol in Gegenwart einer Base bekannt. Die Verfahren wurden allesamt bei Raumtemperatur durchgeführt. Die Reaktionszeit liegt bei 8-18 Stunden.

DE-A 39 36 823 beschreibt die Umsetzung von Oxiran II mit Natrium-1,2,4-triazolid on 5 h bei 75°C. Als Lösungsmittel kommen Dimethylformamid und N-Methylpyrrolidon zur Anwendung.

Die Aufarbeitung der enthaltenen Triazolierungsprodukte erfolgt durch Fällung mit Wasser und/oder Extraktion.

Die Verfahren des Standes der Technik sind mit einer Reihe von Nachteilen behaftet.

Bei der Triazolierung von Verbindungen der Formel II entstehen neben den gewünschten 1- auch 4-substituierte Triazole in Anteilen von 10 - 35 %.

Weiterhin entstehen durch Solvolyse und Ringöffnungsreaktionen eine Reihe von Nebenprodukten, die die Ausbeute verringern und die Isolierung und Reinigung der gewünschten Triazolylmethyloxirane erheblich erschweren.

Zur Reinigung der anfallenden Isomerengemische werden genannt:

Extraktion (z.B. DE-A 3218130, DE-A 3536529, DE-A 380537.6, DE-A 3737888, EP-A 330132, US 4,906,652), Fällung (z.B. DE-A 3936823), Chromatografie (z.B. DE-A 3806089, Rekristallisation aus Diisopropylether (DE-A 3936823, US 4,906,652), Methyltert. butylether/n-Hexan (DE-A 3805376, EP-A 330132), Methyltert. butylether (DE-A 3737888). In allen Fällen müssen verschiedene Methoden kombiniert werden.

Die Reinheit der biologisch wirksamen Isomeren ist überwiegend unter 92 %, nur nach vorbeschriebener komplizierter Aufarbeitung kann man vertretbare Gehalte von über 94 % erreichen.

Aus der Literatur ist weiterhin bekannt, dass bei der Alkylierung von 4-Aminotriazolen quarternäre Triazoliumsalze IV gebildet werden, deren acyclische Aminogruppe analog zur Chemie von entsprechend 1,1-substituierten Hydrazinderivaten beispielsweise mit Natriumnitrit und HCl deaminiert werden können. Es entstehen regioselektiv substituierte Triazolderivate (Houben-Weyl, E 14,479ff).

Diese Reaktion läßt sich auch auf die Alkylierung mit Halomethylketonen übertragen (Astleford et al. J. Org. Chem. 54,731 (1989) und ist für die Herstellung von antimykotischen Wirkstoffen beschrieben, z.B. Can. Pat 2.051.281).

Oxirane reagieren nach dem EP 618.198 unter Öffnung des Oxiranringes zu 2-Hydroxyalkyl-4-Aminotriazoliumsalzen, die zwar deaminiert werden können, aber dann zu 2-Hydroxyalkyltriazolen führen.

Nach dem dargestellten Stand der Technik war deshalb nicht zu erwarten, dass sich Verbindungen der Formel II mit 4-Aminotriazolen zu 4-Aminotriazoliumsalzen umsetzen lassen, in denen der Oxiranring erhalten bleibt.

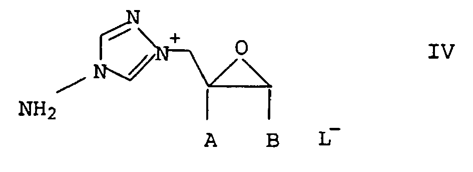

Überraschenderweise konnte nun ein solches Verfahren zur Herstellung von 1-substituierten Triazolylmethyloxiranen dadurch gefunden werden, dass man sterisch gehinderte Oxirane II benutzt und mit 4-Aminotriazolen ohne bzw. in Gegenwart von Katalysatoren oder Hilfsstoffen zu einem quarternären Ammoniumsalz IV umgesetzt und anschließend die nicht alkylierte 4-Aminogruppe einer Deaminierung mit Alkalinitriten und einer Säure oder organischen Nitriten unterzieht. Dabei bildet sich die gewünschten 1-substituierte Triazolylmethyloxirane ohne Anteile von Verunreinigungen oder 4-substituierten Triazolylmethyloxiranen. Die einwandfreie Bildung der erfindungsgemäßen Produkte war nicht erwartet worden, da man erstens eine Reaktion des Aminotriazoles mit dem Oxiranring zu Hydroxyalkyltriazolen erwarten und zweitens auch die Öffnung des Oxiranringes in Gegenwart einer starken Säure befürchten mußte.

Im folgenden wird das erfindungsgemäße Verfahren näher erläutert.

Für das erfindungsgemäße Verfahren eignen sich Azolylmethyl-oxirane, die aus folgenden Ausgangsmaterialien hergestellt worden sind.
a) Oxirane der Formel II in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann und L für eine nukleophil substituierbare Abgangsgruppe steht. Die Oxirane lassen sich wie in der EP-A 94564, US 4,906,652, EP-A 330132, EP-A 334035 und DE 3936823 beschrieben herstellen.
   Bevorzugte Ausgangsmaterialien tragen die folgenden Substituenten, wobei die Bevorzugung jeweils für sich allein oder in Kombination zu sehen ist:
   A und B bedeuten vorzugsweise einen durch Halogen, C₁-C₄-Alkyl oder C₁-C₄-Alkyloxy substituierten Phenylrest.
   Besonders bevorzugt bedeutet A 4-Fluorphenyl und B 2-Chlorphenyl.
   L steht für eine nukleophil substituierte Abgangsgruppe wie beispielsweise Halogenid, Alkylsulfonat, Arylsulfonat oder Alkylsulfat. Vorzugsweise bedeutet L Chlorid, Bromid, Tosylat und Mesylat. Besonders bevorzugt bedeutet L Mesylat.
b) 4-Amino-1,2,4-triazole der Formel III oder analoge Derivate.

Das erfindungsgemäß verwendete 4-Aminotriazol ist leicht aus Hydrazin und Formamid zugänglich (Houben-Weyl E 14,525).

Die Herstellung der erfindungsgemäßen 4-Aminotriazoliumsalze IV erfolgt üblicherweise in Gegenwart eines organischen Lösungsmittels und gegebenenfalls unter Zusatz eines Katalysators oder eines Hilfstoffes bei Temperaturen zwischen 0-150°C, bevorzugt 50-150°C.

Zu den bevorzugten organischen Lösungsmitteln gehören Alkohole wie Methanol, Ethanol, Butanole, Isopropanol, Pentanole, Hexanole, Octanole, Decanole, methylglykol, Ethylglykol, n-Butylglykol, Ketone, wie Aceton, Methylethylketon oder Cyclohexanon, Nitrile, wie Acetonitril oder Propionitril, Ester wie Ethylacetat, Butylacetat, organische Carbonate wie Dimethylcarbonat oder Diethylcarbonat, nichtaromatische und aromatische Kohlenwasserstoffe wie Cyclohexan, Toluol, Chlorbenzol oder 1,2-Dichlorbenzol, Ether wie Tetrahydrofuran, Dimethoxyethan, Dioxan, Amide wie Dimethylformamid, Dimethylacetamid, N-Methylpyrrolidon, Tetramethylharnstoff, ferner Dimethylsulfoxid, Sulfolan und entsprechende Gemische.

Als bevorzugte organische Lösungsmittel kommen Alkohole wie Methanol, Ethanol, isomere Butanole und Pentanole, Isopropanol, 2-Ethylhexanol, Methylglykol, Ethylglykol, n-Butylglykol sowie deren Gemische mit Toluol, in Betracht.

Besonders bevorzugt sind n-Butylglykol, 2-Ethylhexanol sowie deren Gemische mit Toluol.

Als Katalysator geeignet sind quarternäre Ammonium- und Phoshoniumsalze wie Tetrabutylammoniumchlorid, Betaine, wie 4-Dimethylsulfoniumphenolat. Als Hilfsstoffe sind spezielle nucleophile Anionen, z.B. Cyanid, Jodid, Fluorid, Amine, wie DABCO, Dimethylaminopyridin, Dimethylcyclohexylamin, Tributylamin, Triethylamin oder DBU geeignet.

Die Katalysatoren werden in Mengen von 0,01-5 mol% bezüglich des Oxirans II verwendet, die Hilfsstoffe in Mengen von 5-300 mol-%.

Die 4-Aminotriazoliumsalze der Formel IV können aus den Reaktionsmischungen durch Kristallisation und/oder Fällung gegebenenfalls bei tiefen Temperaturen unter 10 °C in reiner Form erhalten werden.

Die 4-Aminotriazoliumsalze der Formel IV werden in Wasser gelöst und mit Alkalimetallnitriten, wie Kalium- oder Natriumnitrit und starken Säuren, wie Salzsäure oder Schwefelsäure bei -10 bis 60°C behandelt. Es können auch organische Nitrite wie beispielsweise n-Butylnitrit oder t-Butylnitrit verwendet werden.

Neben der wässrigen Lösung kann die Deaminierung auch in wässrig/organischen Lösungsmittelgemischen wie Wasser/THF, Wasser/Alkohole oder Wasser/NMP durchgeführt werden.

Weiterhin ist es möglich,die benutzten Lösungsmittel durch Verdampfen zu entfernen und den Rückstand unter Umständen nach einer Abtrennung der nicht wasserlöslichen Komponenten einer Deaminierung zu unterwerfen.

Eine spezielle Variante ist die Durchführung der Aminotriazolierung in einem mit Wasser wenig mischbaren Lösungsmittel wie n-Butanol, i-Pentanol, 2-Ethylhexanol oder Tetrabutylharnstoff, unter gleichzeitiger oder nachfolgender Extraktion des quarternären Salzes mit Wasser.

Die gebildeten Triazolylmethyloxirane fallen in der Regel schon bei der Deaminierung aus der wässrigen Lösung aus. Die Fällung kann durch Neutralisation komplettiert werden.

Durch das erfindungsgemäße Verfahren wird das Verhältnis von 1-zu 4-substituierten Triazolen (Regioselektivität) auf einen Wert von über 50 angehoben. Häufig sind 4-substituiertes Triazolderivate nicht mehr nachweisbar.

Das erhaltene Produkt braucht nicht mehr aufwendig gereinigt zu werden. Der Gehalt an 1-substituierten Triazol liegt in der Regel über 98 %.

Der Anteil an inaktiven Isomeren und Nebenprodukten ist erheblich gesunken und somit die ökologische Effizienz des agrochemisches Wirkstoffes wesentlich erhöht.

Weiterer Gegenstand der vorliegenden Erfindung sind 4-Amino-1,2,4-triazoliumsalze der Formel IV, 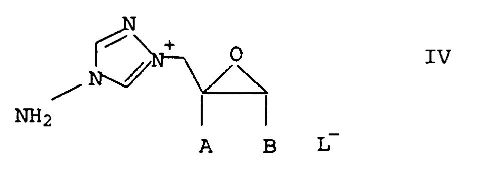 in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, und in der L- für das Anion einer nucleophil substituierbaren Abgangsgruppe steht wie beispielsweise Halogenid, Alkylsulfonat, Arylsulfonat oder Alkylsulfat.

A und B bedeuten vorzugsweise einen durch Halogen, C₁-C₄-Alkyl oder C₁-C₄-Alkyloxy substituierten Phenylrest.

Besonders bevorzugt bedeutet A 4-Fluorphenyl und B - 2-Chlorphenyl.

Bevorzugt steht L- für Chlorid, Bromid Tosylat und Mesylat.

Besonders bevorzugt bedeutet L- Mesylat.

Die für die Substituenten A, B und L genannten organischen Molekülteile stellen Sammelbegriffe für individuelle Aufzählungen der einzelnen Gruppenmitglieder dar. Sämtliche Kohlenwasserstoffketten, also alle Alkyl, Alkoxy, Halogenalkyl, Phenylalkyl, Cycloalkyl, Cycloalkenyl können geradkettig oder verzweigt sein.

Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: z.B. Methyl, Ethyl, Propyl, 1-Methylethyl, Butyl, 1-Methylpropyl, 2-Methylpropyl oder 1,1-Dimethylethyl;
- C₁-C₂-Halogenalkyl: einen C₁-C₂-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlormethyl, Dichlorfluormethyl, Chlordifluormethyl, 2-Fluorethyl, 2-Chlorethyl, 2-Bromethyl,2-Iodethyl, 2,2-Difluorethyl, 2,2,2-Trifluorethyl, 2-Chlor-2-fluorethyl, 2-Chlor-2,2-difluorethyl, 2,2-dichlor-2-fluorethyl, 2,2,2-Trichlorethyl, Pentafluorethyl.
- C₁-C₄-Alkoxy: z.B. Methoxy, Ethoxy, Propoxy, 1-Methylethoxy, Butoxy, 1-Methylpropoxy, 2-Methylpropoxy oder 1,1-Dimethylethoxy;
- Phenyl - C₁-C₂-Alkyl: durch einen Phenylrest substituierten C₁-C₂-Alkyl, wie Benzyl, 1-Phenylethyl und 2-Phenylethyl;
- C₃-C₆-Cycloalkyl: z.B. Cyclopropyl, Cyclobutyl, Cyclopentyl oder Cyclohexyl;
- C₃-C₆-Cycloalkenyl: wie C₃-C₆-Cycloalkyl mit einer Doppelbindung wie Cyclopropenyl, Cyclobutenyl, Cyclopentenyl oder Cyclohexenyl..

Das erfindungsgemäße Verfahren wird durch die folgenden Beispiele erläutert.

Als Oxiran II wurde in allen Fällen eine Verbindung der Formel II mit folgenden Substituenten eingesetzt: L = CH₃SO₂O-, B = 4-Fluorphenyl und A = 2-Chlorphenyl.

### Beispiel 1:

142,8 g der Verbindung II (L = MeSO₂O-, cis/trans 5:95) werden mit 33,6 g 4-Amino-1,2,4-triazol und 400 ml n-Butanol 8 h auf 100 °C erhitzt. Das gebildete Aminotriazoliumsalz fällt aus der Reaktionsmischung in fester Form aus. Der Umsatz bezüglich Mesylat beträgt über 90 % (HPLC-Methode). Nach dem Abkühlen der Mischung können 110 g 4-Aminotriazoliumsalz IV abgetrennt werden (62 % Ausbeute). Die Mutterlauge kann mit dem in Lösung verbliebenen Aminotriazoliumsalz (ca. 45 g) für einen weiteren Ansatz benutzt werden.

Das 4-Aminotriazoliumsalz hat einen Fp. = 192 °C.

### Beispiel 2:

142,8 g der Verbindung II (L = MeSO₂O-, cis/trans 5:95) werden in 500 ml Isopropanol gelöst und anschließend 8 h bei 80 °C mit 33,6 g 4-Aminotriazol erhitzt. Man erreicht einen Umsatz der Verbindung II von 51 % und kann aus der abgekühlten Lösung ca. 72 g 4-Aminotriazoliumsalz IV abtrennen (80 % der umgesetzten Verbindung II). Der Fp. liegt bei 193 °C.

Ein analoger Versuch in Gegenwart von 0,2 g Kaliumiodid erreicht nach 8 h einen Umsatz von 67 %.

### Beispiel 3:

454 ml Mesylat-DMF Lösung, enthaltend 143 g Mesylat II (cis/trans 5:95) werden mit 33,6 g 4-Amino-1,2,4-triazol und 400 ml N-Methylpyrrolidon 2 h auf 130 °C erhitzt. Das gebildete Aminotriazoliumsalz IV kann nach dem Entfernen des Lösungsmittels mit Vakuum durch vorsichtiges Waschen des Rückstandes mit Aceton/MeOH gereinigt werden. Der Umsatz bezüglich Mesylat beträgt über 97 % (HPLC-Methode). Es können 140 g 4-Aminotriazoliumsalz IV isoliert werden (81 % Ausbeute). Das 4-Aminotriazoliumsalz IV hat einen Fp. = 190 °C.

### Beispiel 4:

Eine Mischung aus 5 g (14 mmol) Mesylat und 1,4 g (17 mmol) 4-Amino-1,2,4-triazol in 20 g (153 mmol) 2-Ethylhexanol wird 16 h bei 80 °C gerührt. Danach werden 50 g VE-Wasser zugegeben und nach 5 min bei 65 °C werden die Phasen getrennt. Ausbeute (quant. HPLC): 85 %

### Beispiel 5:

Eine Mischung aus 5 g (14 mmol) Mesylat und 1,1 g (13 mmol) 4-Amino-1,2,4-triazol in 20 g (148 mmol) Diglyme wird 7 h bei 80 °C gerührt. Danach werden 50 g VE-Wasser zugegeben und nach 5 min bei 65 °C werden die Phasen getrennt. Ausbeute (quant. HPLC): 25 %

### Beispiel 6:

Eine Mischung aus 5 g (14 mmol) Mesylat und 1,4 g (17 mmol) 4-Amino-1,2,4-triazol in 20 g (202 mmol) N-Methylpyrrolidon wird 7 h bei 100 °C gerührt. Danach werden 50 g VE-Wasser zugegeben und nach 5 min bei 65 °C werden die Phasen getrennt. Ausbeute (quant. HPLC): 72 %

### Beispiel 7:

Eine Mischung aus 5 g (14 mmol) Mesylat und 1,4 g (17 mmol) 4-Amino-1,2,4-triazol in 20 g (149 mmol) Diethylenglykol-dimethylether wird 7 h bei 100 °C gerührt. Danach werden 50 g VE-Wasser zugegeben und nach 5 min bei 65 °C werden die Phasen getrennt. Ausbeute (quant. HPLC): 51 %

### Beispiel 8:

Eine Mischung aus 5 g (14 mmol) Mesylat und 1,4 g (17 mmol) 4-Amino-1,2,4-triazol in 20 g (203 mmol) Cyclohexanon wird 6 h bei 90 °C gerührt. Danach werden 50 g VE-Wasser zugegeben und nach 5 min bei 65 °C werden die Phasen getrennt. Ausbeute (quant. HPLC): 2 %

### Beispiel 9:

Eine Mischung aus 5 g (14 mmol) Mesylat und 1,4 g (17 mmol) 4-Amino-1,2,4-triazol in 20 g (153 mmol) 1-Octanol wird 6 h bei 80 °C gerührt. Danach werden 50 g VE-Wasser zugegeben und nach 5 min bei 65 °C werden die Phasen getrennt. Ausbeute (quant. HPLC): 65%

### Beispiel 10:

Eine Mischung aus 5 g (14 mmol) Mesylat und 1,4 g (17 mmol) 4-Amino-1,2,4-triazol in 20 g (227 mmol) Ethylencarbonat wird 6 h bei 80 °C gerührt. Danach werden 50 g VE-Wasser zugegeben und nach 5 min bei 65 °C werden die Phasen getrennt. Ausbeute (quant. HPLC): 27 %

### Beispiel 11:

Eine Mischung aus 5 g (14 mmol) Mesylat und 1,4 g (17 mmol) 4-Amino-1,2,4-triazol in 20 g (194 mmol) Benzonitril wird 6 h bei 80 °C gerührt. Danach werden 50 g VE-Wasser zugegeben und nach 5 min bei 65 °C werden die Phasen getrennt. Ausbeute (quant. HPLC): 49 %

### Beispiel 12:

Eine Mischung aus 5 g (14 mmol) Mesylat und 1,4 g (17 mmol) 4-Amino-1,2,4-triazol in 20 g (200 mmol) Cyclohexanol wird 17 h bei 80 °C gerührt. Danach werden 50 g VE-Wasser zugegeben und nach 5 min bei 65 °C werden die Phasen getrennt. Ausbeute (quant. HPLC): 29 %

### Beispiel 13:

Eine Mischung aus 5 g (14 mmol) Mesylat und 1,4 g (17 mmol) 4-Amino-1,2,4-triazol in 20 g (136 mmol) 1,2-Dichlorbenzol wird 16 h bei 80 °C gerührt. Danach werden 50 g VE-Wasser zugegeben und nach 5 min bei 65 °C werden die Phasen getrennt. Ausbeute (quant. HPLC): 21 %

### Beispiel 14:

Zu einer Lösung aus 252,2 g (3,0 mol) 4-Amino-1,2,4-triazol in 1070,4 g (8,4 mol) n-Butylglykol werden bei 90 °C und 130 mbar 356,8 g (1,0 mol) Mesylat in 2020 g Toluol zudosiert. Dosierung und Destillation werden nach minimal 6 h beendet und anschließend auf 85 °C abkühlen gelassen. Danach wird bis 65 °C eine Rampe von 3 K/h gefahren. Nach Abkühlung der erhaltenen Maische auf 25 °C wird die Suspension anschließend über eine Saugfritte filtriert. Ausbeute (quant. HPLC): 98 %

### Beispiel 15:

Zu einer Lösung aus 86,4 g (1,03 mol) 4-Amino-1,2,4-triazol und 128,3 g Tri-n-butylamin (0,69 mol) in 600g g (4,7 mol) n-Butylglykol werden bei 90 °C und 130 mbar 121 g (0,34 mol) Mesylat in 679 g Toluol zudosiert. Dosierung und Destillation von 556 g werden nach minimal 6 h beendet und anschließend auf 85 °C abkühlen gelassen. Danach wird bis 65 °C eine Rampe von 3 K/h gefahren. Nach Abkühlung der erhaltenen Maische auf 25 °C wird die Suspension anschließend über eine Saugfritte filtriert. Ausbeute (quant. HPLC): 77 %

### Beispiel 16:

Zu einer Lösung aus 86,4 g (1,03 mol) 4-Amino-1,2,4-triazol und 43,2 g Tri-n-butylamin (0,34 mol) in 600g g (4,7 mol) n-Butylglykol werden bei 90 °C und 130 mbar 121 g (0,34 mol) Mesylat in 679 g Toluol zudosiert. Dosierung und Destillation von 556 g werden nach minimal 6 h beendet und anschließend auf 85 °C abkühlen gelassen. Danach wird bis 65 °C eine Rampe von 3 K/h gefahren. Nach Abkühlung der erhaltenen Maische auf 25 °C wird die Suspension anschließend über eine Saugfritte filtriert. Ausbeute (quant. HPLC): 52 %

### Beispiel 17:

Zu einer Lösung aus 84,8 g (1,01 mol) 4-Amino-1,2,4-triazol in 600 g (4,7 mol) n-Butylglykol werden bei 90 °C und 130 mbar 128,2 g (0,36 mol) Mesylat in 725 g Toluol zudosiert. Dosierung und Destillation von 631 g Toluol werden nach minimal 6 h beendet und anschließend auf 85 °C abkühlen gelassen. Danach wird bis 65 °C eine Rampe von 3 K/h gefahren. Nach Abkühlung der erhaltenen Maische auf 25 °C wird die Suspension anschließend über eine Saugfritte filtriert. Ausbeute (quant. HPLC): 99 %

### Deaminierung:

### Beispiel 18:

### Durchführung

50 mMol festes 4-Aminotriazoliumsalz IV (22 g, A = 4-Fluorphenyl und B = 2-Chlorphenyl) werden in 150 ml Wasser aufgenommen und mit 110 mMol konz. Salzsäure (11 ml) versetzt. Anschließend wird auf 0 °C abgekühlt. Man läßt eine Lösung von 3,6 g (52 mMol) Natriumnitrit in 50 ml Wasser bei dieser Temperatur langsam zutropfen, wobei eine schonende Gasentwicklung einsetzt. Nach beendeter Zugabe läßt man auf Raumtemperatur erwärmen und neutralisiert anschließend mit verdünnter Kaliumcarbonat-Lösung (- 50 ml einer 15 %igen wässrigen Lösung). Das ausfallende Produkt wird abgesaugt, mit Wasser gewaschen und getrocknet.

Ausbeute: 95 %, Fp. 136 °C, Gehalt: 98,5 %b trans-Epoxiconazol (trans bez. A und B).

### Beispiel 19:

Man legt unter Stickstoff 1162,5 g (0,5 mol) 18,9 %ige wässrige Triazoliumsalzlösung vor und stellt den pH mittels 18 %iger Salzsäure auf < 1,0 ein. Die Reaktionsmischung wird auf 60 °C aufgeheizt. Anschließend werden parallel 250 ml (0,8 mol) 20 %ige Natriumnitrit-Lösung (1,6 eq) und 80 g (0,39 mol)18 %ige Salzsäure (0,8 eq) bei pH 1 über einen Zeitraum von 1 h zudosiert. Die Suspension wird 1,5 h bei 60 °C nachgerührt, auf 20 °C abgekühlt und mit 15 %iger NaOH neutralisiert. Danach wird die Suspension über eine Saugfritte abgetrennt und der Feststoff im VTS bei 20 mbar und 50 °C Innentemperatur getrocknet. Ausbeute (quant. HPLC): 87.8 %

### Beispiel 20:

Man legt unter Stickstoff 214 g (0,05 mol) 10,8%ige wässrige Triazoliumsalzlösung vor. Die Reaktionsmischung wird auf 50 °C aufgeheizt. Anschließend werden 7,0 g (0,065 mol) n-Butylnitrit über einen Zeitraum von 1 h zudosiert. Die Suspension wird 1 h bei 50 °C nachgerührt, auf 20 °C abgekühlt und über eine Saugfritte abgetrennt und der Feststoff im VTS bei 20 mbar und 50 °C Innentemperatur getrocknet. Ausbeute (quant. HPLC): 49,0 %

### Beispiel 21:

Man legt unter Stickstoff 940 g (0,13 mol) 6,1%ige wässrige Triazoliumsalzlösung vor und stellt den pH mittels 18 %iger Salzsäure auf < 1,0 ein. Die Reaktionsmischung wird auf 60 °C aufgeheizt. Anschließend werden parallel 61,3 ml (0,2 mol) 20 %ige Natriumnitrit-Lösung (1,5 eq) und 29 g (0,39 mol) 18 %ige Salzsäure (1,1 eq) bei pH 1 über einen Zeitraum von 1 h zudosiert. Die Suspension wird 1,5 h bei 60 °C nachgerührt, auf 20 °C abgekühlt und über eine Saugfritte abgetrennt und der Feststoff im VTS bei 20 mbar und 50 °C Innentemperatur getrocknet. Ausbeute (quant. HPLC): 92,7 %

### Beispiel 22:

### Extraktion

142,8 g der Verbindung II werden in 500 ml n-Butanol gelöst und anschließend 7 h mit 33,6 g 4-Aminotriazol erhitzt. Man erreicht einen Umsatz der Verbindung II von 83 %. Nach der Zugabe von 100 ml Toluol extrahiert man die organische Phase dreimal mit jeweils 500 ml Wasser. Die wässrige Lösung wird auf 0 - 5 °C gekühlt, mit 160 ml 18 % HCl acidifiziert und anschließend portionsweise mit einer Lösung von 27,6 g Natriumnitrit in 100 ml Wasser versetzt (N₂O-Entwicklung). Dabei fällt ein weißer Niederschlag aus, der nach dem Waschen mit MeOH/Wasser und Trocknen bei 80 °C 65 g isomerenfreies trans-Epoxiconazol ergibt. Aus der Mutterlauge können nach dem Neutralisieren mit 2N NaOH noch etwa 18 g unreineres Epoxiconazol erhalten werden.

Ausbeute an reinem Produkt bezüglich der Verbindung II: 59,3%, gehalt: 97,9 % trans-Epoxiconazol,Fp. von 136 °C.

Die organische Phase kann für einen weiteren Ansatz benutzt werden, wobei man das zugegebene Toluol durch Abdestillieren zurückgewinnt und erst bei der Extraktion wieder zusetzt.

### Beispiel 23:

142,8 g der Verbindung II werden in 500 ml n-Butanol gelöst und anschließend 12 h mit 33,6 g 4-Aminotriazol erhitzt. Man erreicht einen Umsatz der Verbindung II von 97 %. Nach dem vorsichtigen Entfernen des Lösungsmittels bei 8 mbar und 60 °C wird der Rückstand in 1000 1 Wasser aufgelöst und zweimal mit 100 ml Toluol nicht umgesetzte Verbindung II extrahiert.

Die wässrige Lösung wird auf 0 - 5 °C gekühlt, mit 160 ml 18 % HCl acidifiziert und anschließend portionsweise mit einer Lösung von 27,6 g Natriumnitrit in 100 ml Wasser versetzt (N₂O-Entwicklung). Dabei fällt ein weißer Niederschlag aus. Nach 4 h wird mit Kaliumcarbonatlösung neutralisiert und abgesaugt. Man erhält nach dem Waschen mit MeOH/Wasser und Trocknen bei 80 °C 143 g isomerenfreies trans-Epoxiconazol.

| | |
|---|---|
| Ausbeute: | 83 % |
| Fp. | 136 °C |
| Gehalt: | 98,7 % |

### Beispiel 24:

### Extraktive Eintopfreaktion

### Durchführung:

35,6 g der Verbindung II (L = MeSO₂O-, cis/trans 5:95, 100 mMol) werden in 200 ml Tetrabutylharnstoff aufgenommen und mit 8,8 g (105 mMol) 4-Amino-1,2,4-triazol sowie 200 ml Wasser unter Zugabe von Tetrabutylammoniumchlorid versetzt. Anschließend wird 4 Stunden am Rückfluss gekocht (Umsatz bez. auf Verbindung II ca. 30 %). Das Reaktionsprodukt geht hierbei in der wässrigen Phase in Lösung, während nicht umgesetzte Alkylverbindung sowie überschüssiges Aminotriazol in der nicht mischbaren, organischen Phase verbleibt. Man läßt abkühlen und trennt die wässrige Phase ab. Die organische Phase kann rückgeführt werden.

Die wässrige Phase wird mit der 2,2fachen molaren Menge an konz. Salzsäure versetzt und auf 0 °C abgekühlt. Man läßt die entsprechende molare Menge an Natriumnitrit, gelöst in 50 ml Wasser, bei dieser Temperatur langsam zutropfen, wobei eine schonende Gasentwicklung einsetzt. Nach beendeter Zugabe läßt man auf Raumtemperatur erwärmen und neutralisiert anschließend mit verdünnter Kaliumcarbonatlösung. Das ausgefallene Produkt wird abgesaugt, mit Wasser gewaschen und getrocknet.

Ausbeute (bezogen auf eingesetzte Alkylverbindung): 8,2 g trans-Epoxiconazol (80 % bez. auf umgesetzte Verbindung II).

## Patentansprüche

1. Verfahren zur Herstellung von 1,2,4-Triazol-1-ylmethyloxiranen der allgemeinen Formel I 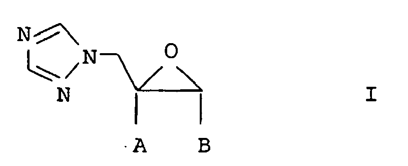 in der A und B gleich oder verschieden sind und unabhängig voneinander C₁-C₄-Alkyl, Phenyl-C₁-C₂-alkyl, C₃-C₆-Cycloalkyl, C₃-C₆-Cycloalkenyl, Tetrahydropyranyl, Tetrahydrofuranyl, Dioxanyl oder Phenyl bedeuten, wobei der Phenylrest ein bis drei Substituenten ausgewählt aus der Gruppe: Halogen, Nitro, C₁-C₄-Alkyl, C₁-C₄-Alkyloxy, Phenoxy, Amino, C₁-C₂-Halogenalkyl oder Phenylsulfonyl tragen kann, **dadurch gekennzeichnet, dass** man
a) ein Oxiran der Formel II, 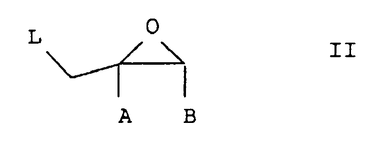 in der A und B die oben genannte Bedeutung besitzen und L für eine nukleophil substituierbare Abgangsgruppe steht, mit 4-Amino-1,2,4-triazol der Formel III 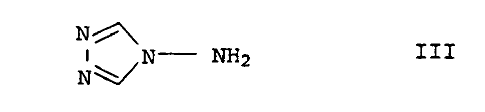 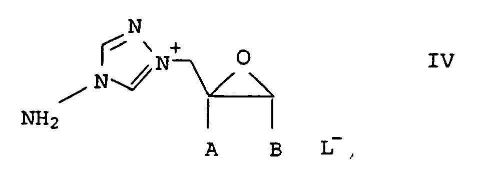 zu 4-Amino-1,2,4-triazoliumsalzen der Formel IV umsetzt und
b) die 4-Amino-1,2,4-triazoliumsalze IV mit Alkalinitriten und Säure oder organischen Nitriten zu 1,2,4-Triazol-1-ylmethyloxiranen der allgemeinen Formel I deaminiert.

2. Verfahren gemäß Anspruch 1, **dadurch gekennzeichnet, dass** man die Umsetzung in der Stufe a) in Gegenwart eines organischen Lösungsmittels durchführt.

3. Verfahren gemäß Anspruch 2, **dadurch gekennzeichnet, dass** man als organisches Lösungsmittel Alkohole, Ketone, Nitrile, Ester, organische Carbonate, nichtaromatische und aromatische Kohlenwasserstoffe, Ether, Amide, Dimethylsulfoxid, Sulfolan oder deren Gemische verwendet.

4. Verfahren gemäß einem der Ansprüche 2 oder 3, **dadurch gekennzeichnet, dass** man als organisches Lösungsmittel Methanol, Ethanol, Butanole, Isopropanol, Pentanole, Hexanole, Octanole, Decanole, Methylglykol, Ethylglykol, n-Butylglykol, Aceton, Methylethylketon, Cyclohexanon, Acetonitril, Propionitril, Ethylacetat, Butylacetat, Tetrahydrofuran, Dimethoxyethan, Dioxan, Dimethylformamid, Dimethylacetamid, N-Methylpyrrolidon, Tetramethylharnstoff, Dimethylsulfoxid, Sulfolan oder deren Gemische verwendet.

5. Verfahren nach Anspruch 4, **dadurch gekennzeichnet, dass** man als organisches Lösungsmittel n-Butylglykol, 2-Ethylhexanol oder deren Gemische mit Toluol verwendet.

6. Verfahren gemäß einem der Ansprüche 1 bis 5, **dadurch gekennzeichnet, dass** man die Umsetzung in der Stufe a) bei Temperaturen von 50 bis 150°C durchführt.

7. Verfahren gemäß einem der Ansprüche 1 bis 6, **dadurch gekennzeichnet, dass** man die Umsetzung in der Stufe a) in Gegenwart von 0,01-5 mol-% eines Katalysators oder 5-300 mol-% eines Hilfsstoffes durchführt.

8. Verfahren gemäß Anspruch 7, **dadurch gekennzeichnet, dass** man als Katalysator quarternäre Ammoniumsalze, quaternäre Phosphoniumsalze, Betaine und/oder als Hilfsstoffe nucleophile Anionen und Amine verwendet.

9. Verfahren gemäß einem der Ansprüche 7 bis 8, **dadurch gekennzeichnet, dass** man als Katalysator Tetrabutylammoniumchlorid, 4-Dimethylsulfoniumphenolat und/oder als Hilfsstoffe Cyanide, Jodide, Fluoride, DABCO, Dimethylaminopyridin, Dimethylcycloheylamin, Tributylamin, Triethylamin oder DBU verwendet.

10. Verfahren gemäß einem der Ansprüche 1 bis 9, **dadurch gekennzeichnet, dass** man die in der Stufe a) gebildeten 4-Aminotriazoliumsalze der Formel IV durch Fällung und/oder Kristallisation aus der Reaktionsmischung abtrennt.

11. Verfahren gemäß Anspruch 10, **dadurch gekennzeichnet, dass** man die Fällung und/oder Kristallisation der 4-Aminotriazolinumsalze der Formel IV bei Temperaturen unter 10°C durchführt.

12. Verfahren gemäß einem der Ansprüche 1 bis 11, **dadurch gekennzeichnet, dass** man die in der Stufe a) gebildeten 4-Aminotriazolumsalze der Formel IV durch kontinuierliche und/oder diskontinuierliche Extraktion aus der Reaktionsmischung extrahiert.

13. Verfahren gemäß Anspruch 12, **dadurch gekennzeichnet, dass** man die kontinuierliche und/oder diskontinuierliche Extraktion mit Wasser, ggf. in Gegenwart eines mit Wasser nicht mischbaren organischen Lösungsmittels, durchgeführt.

14. Verfahren gemäß einem der Ansprüche 1 bis 13, **dadurch gekennzeichnet, dass** man die Deaminierung der Stufe b) in wässriger Lösung, Wasser/THF, Wasser/Alkohole oder Wasser/NMP durchgeführt.

15. Verfahren gemäß einem der Ansprüche 1 bis 14, **dadurch gekennzeichnet, dass** man die Deaminierung der Stufe b) mit organischen Nitriten in wässriger oder organischer Lösung oder in wässrig/organischen Lösungsgemischen wie Wasser/THF, Wasser/Alkohole, Wasser/NMP durchführt.

16. Verfahren gemäß einem der Ansprüche 14 oder 15, **dadurch gekennzeichnet, dass** man die Deaminierung der Stufe b) bei einer Temperatur von -10 bis 60°C durchführt.

17. 4-Amino-1,2,4-triazoliumsalze der Formel IV, 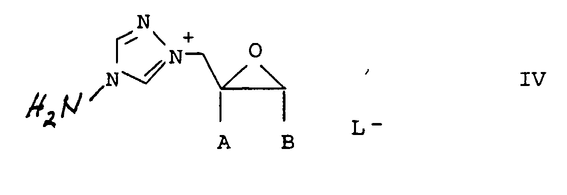 in der A, B und L- die in Anspruch 1 gegebene Bedeutung haben.

18. 4-Amino-1,2,4-triazoliumsalze der Formel IV gemäß Anspruch 17, in der A und B gleich oder verschieden sind und einen durch Halogen, C₁-C₄-Alkyl oder C₁-C₄-Alkoxy substituierten Phenylrest bedeuten.

19. 4-Amino-1,2,4-triazoliumsalze der Formal IV gemäß Anspruch 17, in der A für 4-Fluorphenyl und B für 2-Chlorphenyl stehen.

## Claims

1. A process for the preparation of 1,2,4-triazol-1-ylmethyloxiranes of the formula I 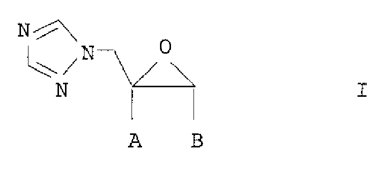 in which A and B are identical or different and, independently of one another, are C₁-C₄-alkyl, phenyl-C₁-C₂-alkyl, C₃-C₆-cycloalkyl, C₃-C₆-cycloalkenyl, tetrahydropyranyl, tetrahydrofuranyl, dioxanyl or phenyl, where the phenyl radical can carry one to three substituents chosen from the group: halogen, nitro, C₁-C₄-alkyl, C₁-C₄-alkyloxy, phenoxy, amino, C₁-C₂-haloalkyl or phenylsulfonyl, which comprises reacting
a) an oxirane of the formula II 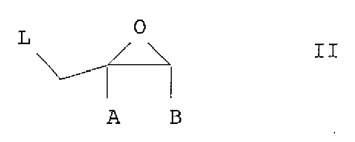 in which A and B have the meanings given above and L is a nucleophilically substitutable leaving group, with 4-amino-1,2,4-triazole of the formula III 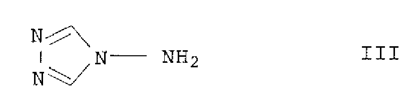 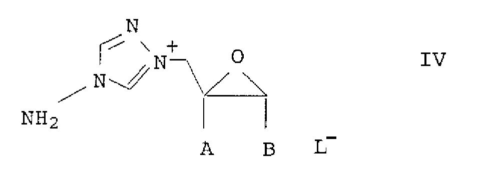 to give 4-amino-1,2,4-triazolium salts of the formula IV and
b) deaminating the 4-amino-1,2,4-triazolium salts IV with alkali metal nitrites and acid or organic nitrites to give 1,2,4-triazol-1-ylmethyloxiranes of the formula I.

2. A process as claimed in claim 1, wherein the reaction in stage a) is carried out in the presence of an organic solvent.

3. A process as claimed in claim 2, wherein alcohols, ketones, nitriles, esters, organic carbonates, nonaromatic and aromatic hydrocarbons, ethers, amides, dimethyl sulfoxide, sulfolane or mixtures thereof are used as organic solvent.

4. A process as claimed in either claim 2 or 3, wherein the organic solvent used is methanol, ethanol, butanols, isopropanol, pentanols, hexanols, octanols, decanols, methyl glycol, ethyl glycol, n-butyl glycol, acetone, methyl ethyl ketone, cyclohexanone, acetonitrile, propionitrile, ethyl acetate, butyl acetate, tetrahydrofuran, dimethoxyethane, dioxane, dimethylformamide, dimethylacetamide, N-methylpyrrolidone, tetramethylurea, dimethyl sulfoxide, sulfolane or mixtures thereof.

5. A process as claimed in claim 4, wherein the organic solvent used is n-butyl glycol, 2-ethylhexanol or mixtures thereof with toluene.

6. A process as claimed in any of claims 1 to 5, wherein the reaction in stage a) is carried out at temperatures of from 50 to 150°C.

7. A process as claimed in any of claims 1 to 6, wherein the reaction in stage a) is carried out in the presence of 0.01-5 mol% of a catalyst or 5-300 mol% of an auxiliary.

8. A process as claimed in claim 7, wherein quaternary ammonium salts, quaternary phosphonium salts and betaines are used as catalyst and/or nucleophilic anions and amines are used as auxiliaries.

9. A process as claimed in any of claims 7 to 8, wherein tetrabutylammonium chloride and 4-dimethylsulfonium phenoxide are used as catalyst and/or cyanides, iodides, fluorides, DABCO, dimethylaminopyridine, dimethylcyclohexylamine, tributylamine, triethylamine or DBU are used as auxiliaries.

10. A process as claimed in any of claims 1 to 9, wherein the 4-aminotriazolium salts of the formula IV formed in stage a) are separated off from the reaction mixture by precipitation and/or crystallization.

11. A process as claimed in claim 10, wherein the precipitation and/or crystallization of the 4-aminotriazolium salts of the formula IV is carried out at temperatures below 10°C.

12. A process as claimed in any of claims 1 to 11, wherein the 4-aminotriazolium salts of the formula IV formed in stage a) are extracted from the reaction mixture by continuous and/or discontinuous extraction.

13. A process as claimed in claim 12, wherein the continuous and/or discontinuous extraction is carried out with water, optionally in the presence of a water-immiscible organic solvent.

14. A process as claimed in any of claims 1 to 13, wherein the deamination in stage b) is carried out in aqueous solution, water/THF, water/alcohols or water/NMP.

15. A process as claimed in any of claims 1 to 14, wherein the deamination in stage b) is carried out with organic nitrites in aqueous or organic solution or in aqueous/organic solvent mixtures such as water/THF, water/alcohols, water/NMP.

16. A process as claimed in either claim 14 or 15, wherein the deamination in stage b) is carried out at a temperature of from -10 to 60°C.

17. A 4-amino-1,2,4-triazolium salt of the formula IV 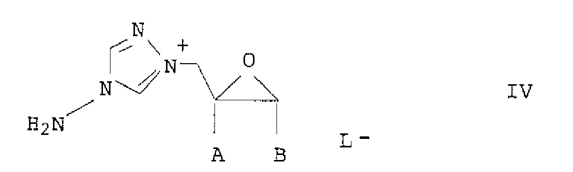 in which A, B and L- have the meanings given in claim 1.

18. A 4-amino-1,2,4-triazolium salt of the formula IV as claimed in claim 17, in which A and B are identical or different and are a phenyl radical substituted by halogen, C₁-C₄-alkyl or C₁-C₄-alkoxy.

19. A 4-amino-1,2,4-triazolium salt of the formula IV as claimed in claim 17, in which A is 4-fluorophenyl and B is 2-chlorophenyl.

## Revendications

1. Procédé de préparation de 1,2,4-triazol-1-ylméthyloxiranes de formule générale I 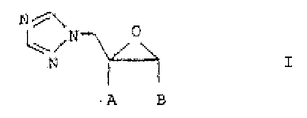 dans laquelle A et B sont identiques ou différents et signifient, indépendamment l'un de l'autre, un alkyle en C₁-C₄, un phényl alkyle en C₁-C₂, un cycloalkyle en C₃-C₆, un cycloalcényle en C₃-C₆, un tétrahydropyranyle, un tétrahydrofuranyle, un dioxanyle ou un phényle, le radical phényle pouvant porter un à trois substituants sélectionnés parmi le groupe des halogène, nitro, alkyle en C₁-C₄, alkyloxy en C₁-C₄, phénoxy, amino, halogénoalkyle en C₁-C₂ ou phénylsulfonyle, **caractérisé en ce qu'**on transforme
a) un oxirane de formule II, 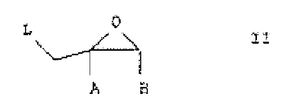 dans laquelle A et B ont la signification donnée ci-dessus et L représente un groupe initial substituable de manière nucléophile, avec un 4-amino-1,2,4-triazole de formule III 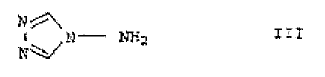 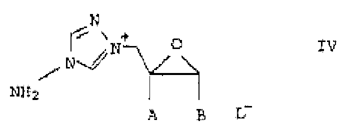 en sels de 4-amino-1,2,4-triazolium de formule IV et
b) on désamine les sels de 4-amino-1,2,4-triazolium IV avec des nitrites alcalins et un acide ou des nitrites organiques en 1,2,4-triazol-1-ylméthyloxiranes de formule générale I.

2. Procédé selon la revendication 1, **caractérisé en ce qu'**on effectue la transformation de l'étape a) en présence d'un solvant organique.

3. Procédé selon la revendication 2, **caractérisé en ce qu'**on utilise comme solvant organique des alcools, des cétones, des nitriles, des esters, des carbonates organiques, des hydrocarbures non aromatiques et aromatiques, des éthers, des amides, du diméthylsulfoxyde, du sulfolane ou leurs mélanges.

4. Procédé selon l'une quelconque des revendications 2 ou 3, **caractérisé en ce qu'**on utilise comme solvant organique du méthanol, de l'éthanol, des butanols, de l'isopropanol, des pentanols, des hexanols, des octanols, des décanols, du méthylglycol, de l'éthylglycol, du n-butylglycol, de l'acétone, de la méthyléthylcétone, de la cyclohexanone, de l'acétonitrile, du propionitrile, de l'éthylacétate, de l'acétate de butyle, du tétrahydrofurane, du diméthoxyéthane, du dioxane, du diméthylformamide, du diméthylacétamide, de la N-méthylpyrrolidone, de la tétraméthylurée, du diméthylsulfoxyde, du sulfolane ou leurs mélanges.

5. Procédé selon la revendication 4, **caractérisé en ce qu'**on utilise comme solvant organique du n-butylglycol, du 2-éthylhexanol ou leurs mélanges avec du toluène.

6. Procédé selon l'une quelconque des revendications 1 à 5, **caractérisé en ce qu'**on effectue la transformation de l'étape a) à des températures comprises entre 50 et 150°C.

7. Procédé selon l'une quelconque des revendications 1 à 6, **caractérisé en ce qu'**on effectue la transformation de l'étape a) en présence de 0,01-5% en mole d'un catalyseur ou de 5-300% en mole d'un adjuvant.

8. Procédé selon la revendication 7, **caractérisé en ce qu'**on utilise comme catalyseur des sels d'ammonium quaternaires, des sels de phosphonium quaternaires, des bétaïnes et/ou comme adjuvants des anions nucléophiles et des amines.

9. Procédé selon l'une quelconque des revendications 7 à 8, **caractérisé en ce qu'**on utilise comme catalyseur du chlorure de tétrabutylammonium, du phénolate de 4-diméthylsulfonium et/ou comme adjuvants des cyanures, des iodures, des fluorures, du DABCO, de la diméthylaminopyridine, de la diméthylcyclohexylamine, de la tributylamine, de la triéthylamine ou du DBU.

10. Procédé selon l'une quelconque des revendications 1 à 9, **caractérisé en ce qu'**on sépare du mélange réactionnel les sels de 4-aminotriazolium formés à l'étape a) et de formule IV par précipitation et/ou cristallisation.

11. Procédé selon la revendication 10, **caractérisé en ce qu'**on effectue la précipitation et/ou la cristallisation des sels de 4-aminotriazolium de formule IV à des températures inférieures à 10°C.

12. Procédé selon l'une quelconque des revendications 1 à 11, **caractérisé en ce qu'**on extrait du mélange réactionnel les sels de 4-aminotriazolium formés à l'étape a) et de formule IV par extraction continue et/ou discontinue.

13. Procédé selon la revendication 12, **caractérisé en ce qu'**on effectue l'extraction continue et/ou discontinue avec de l'eau, le cas échéant en présence d'un solvant organique non miscible à l'eau.

14. Procédé selon l'une quelconque des revendications 1 à 13, **caractérisé en ce qu'**on effectue la désamination de l'étape b) en solution aqueuse, eau/THF, eau/alcools ou eau/NMP.

15. Procédé selon l'une quelconque des revendications 1 à 14, **caractérisé en ce qu'**on effectue la désamination de l'étape b) avec des nitrites organiques en solution aqueuse ou organique ou dans des mélanges de solvants aqueux/organiques comme eau/THF, eau/alcools ou eau/NMP.

16. Procédé selon l'une quelconque des revendications 14 ou 15, **caractérisé en ce qu'**on effectue la désamination de l'étape b) à une température comprise entre -10 et 60°C.

17. Sels de 4-amino-1,2,4-triazolium de formule IV, 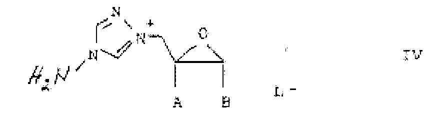 dans laquelle A, B et L'ont la signification donnée à la revendication 1.

18. Sels de 4-amino-1,2,4-triazolium de formule IV selon la revendication 17, dans laquelle A et B sont identiques ou différents et signifient un radical phényle substitué par un halogène, un alkyle en C₁-C₄ ou un alcoxy en C₁-C₄.

19. Sels de 4-amino-1,2,4-triazolium de formule IV selon la revendication 17, dans laquelle A représente du 4-fluorophényle et B du 2-chlorophényle.
